# EUROPEAN PATENT APPLICATION

(11) **EP 4 620 396 A1**
(43) Date of publication of application: **24.09.2025**
(21) Application number: 23891866.8
(22) Date of filing: 30.10.2023
(51) Int. Cl.: A61B 6/00, A61B 6/03, A61B 5/055, G16H 50/20, G16H 30/40, G06N 3/08

(54) **METHOD OF PROVIDING INFORMATION ON RIB FRACTURE, AND DEVICE USING SAME**

(30) Priority: 16.11.2022 KR 20220153263
(71) Applicant: INDUSTRY-ACADEMIC COOPERATION FOUNDATION, YONSEI UNIVERSITY, Seoul 03722 (KR)
(72) Inventor: KIM, Sungjun, Seoul 06004 (KR); YOON, Youngno, Goyang-si Gyeonggi-do 10413 (KR)
(74) Representative: Müller Schupfner & Partner Patent- und Rechtsanwaltspartnerschaft mbB (Muc)
(86) International application number: PCT/KR2023/017005
(87) International publication number: WO 2024/106799

(57) **Abstract**

Provided are a method of providing information on a rib fracture, and a device for providing information on a rib fracture by using same. The method of providing information on a rib fracture, implemented by a processor, comprises the steps of: receiving a chest medical image of a subject, extracting the chest medical image patch by patch, determining by using a convolutional neutral network model pretrained to determine rib fracture probability by using a patch as an input, rib fracture probability by using the patch as an input; generating a class activation map (CAM) patch by patch, and reconstructing the entire class activation map generated patch by patch, to correspond to the entire chest medical image.

## Description

### Technical Field

The present disclosure relates to a method of providing information on rib fractures and a device using the same.

### Background Art

Diagnosis of rib fractures may be made through X-rays. However, the diagnosis of fractures based on the X-ray images may be ambiguous or difficult to accurately confirm the fracture pattern, so additional special tests such as computed tomography and magnetic resonance imaging may be required. Furthermore, since medical staff directly determine the presence or absence of fractures with their own eyes, there may be limitations in providing reliable diagnostic results.

Meanwhile, deep learning learns a very large amount of data, and when new data is input, it selects the answer with the highest probability based on the learning results. There is an increasing trend of attempts to use artificial intelligence to diagnose fractures using deep learning.

The background technology of the disclosure has been written to facilitate understanding of the present disclosure. It should not be understood that the matters described in the background technology of the disclosure are recognized as prior art.

### Detailed Description of the Invention

### Technical Problem

Accidents involving the chest, where the heart, lungs, liver, and the like are located in the rib area, are often critical. The inventors of the present disclosure have recognized that when medical staff examine the chest using chest medical imaging to determine if there is a problem with the chest, they often overlook and miss rib fractures that have a relatively low risk.

Meanwhile, the chest ribs are composed of 24 bones, 12 on each left and right. Determining whether or not there is a chest rib fracture requires the experience, time, and attention of the medical staff.

Accordingly, the inventors of the present disclosure, as a means to solve the aforementioned problem, have sought to develop a method and device capable of quickly analyzing with high accuracy the points expected to be rib fractures and the rib fracture probability at the corresponding points when taking chest medical images for lesions other than rib fractures using an artificial intelligence model, and of visually displaying the points on chest medical images to assist in the diagnosis and treatment of rib fractures.

As a result, the inventors of the present disclosure developed a method of providing information on a rib fracture based on a class activation map that visually indicates the point expected to be the rib fracture, and a device for providing information on rib fractures using the same.

Accordingly, an object of the present disclosure is to provide a method of providing information on a rib fracture that visually indicates a point expected to be a rib fracture and a rib fracture probability at the corresponding point on a chest medical image, and a device using the same.

Objects of the present disclosure are not limited to the objects mentioned above, and other objects not mentioned will be clearly understood by those skilled in the art from the description below.

### Technical Solution

In order to solve the above-described problem, a method of providing information on a rib fracture according to one embodiment of the present disclosure is provided. The method of providing information on the rib fracture according to one embodiment of the present disclosure is implemented by a processor, and includes: receiving a chest medical image of a subject; extracting the chest medical image patch by patch; determining a rib fracture probability with a patch as input, using a convolutional neutral network model pretrained to determine the rib fracture probability by using the patch as an input; generating a class activation map (CAM) patch by patch; and reconstructing the entire class activation map generated patch by patch, to correspond to the entire chest medical image.

According to a feature of the present disclosure, the extracting of the chest medical image pitch by patch may further include extracting a patch of a predetermined size while moving the patch in a sliding window manner.

According to another feature of the present disclosure, the determining of the rib fracture probability may further include determining, when extraction areas of the patches overlap each other, an average value or maximum value of the rib fracture probabilities of the overlapping patches as the rib fracture probability for each patch.

According to still another feature of the present disclosure, the determining of the rib fracture probability may further include classifying the class activation map into a class in which there is a rib fracture when the rib fracture probability is equal to or more than a predetermined value, and classifying the class activation map into a class in which there is no rib fracture when the rib fracture probability is less than the predetermined value.

According to still another feature of the present disclosure, the reconstructing of the entire class activation map so that the class activation map generated patch by patch corresponds to the entire chest medical image may include reconstructing, when reconstructing, only the class activation map classified as the class in which there is the rib fracture.

According to still another feature of the present disclosure, the generating of the class activation map patch by patch may further include overlapping the class activation maps each other, and determining an average value of activation degrees for pixels of the overlapping class activation maps as an activation degree of the pixel of the entire class activation map.

According to still another feature of the present disclosure, the method may further include overlapping and displaying the rib fracture probability for each patch and the reconstructed entire class activation map with the entire chest medical image.

According to still another feature of the present disclosure, the convolutional neural network model may include one or more convolutional neural network models selected from ResNet, EfficientNet, VGGNet, GoogLeNet, LeNet, AlexNet, U-Net, OverFeat, DenseNet, and ZFNet.

According to still another feature of the present disclosure, the chest medical image may be at least one selected from an X-ray image, computed tomography (CT), magnetic resonance imaging (MRI), and positron emission tomography (PET).

In order to solve the problem as described above, a device for providing information on a rib fracture according to another embodiment of the present disclosure is provided. The device for providing information on a rib fracture includes: a communication unit configured to receive a chest medical image of a subject; and a processor connected to communicate with the communication unit, in which the processor is configured to extract the chest medical image patch by patch, determine rib fracture probability patch by patch by inputting the extracted chest medical image to a pretrained convolutional neural network model, generate a class activation map (CAM) patch by patch, and reconstruct the entire class activation map generated patch by patch, to correspond to the entire chest medical image.

According to a feature of the present disclosure, the processor may be configured to extract a patch of a predetermined size while moving the patch in a sliding window manner.

According to another feature of the present disclosure, the processor may be further configured to determine, when extraction areas of the patches overlap each other, an average value or maximum value of the rib fracture probabilities of the overlapping patches as the rib fracture probability for each patch.

According to still another feature of the present disclosure, the processor may be further configured to classify the class activation map into a class in which there is a rib fracture when the rib fracture probability is equal to or more than a predetermined value, and classify the class activation map into a class in which there is no rib fracture when the rib fracture probability is less than the predetermined value.

According to still another feature of the present disclosure, the processor may be further configured to reconstruct only the class activation map classified as the class in which there is the rib fracture when reconstructing to correspond to the entire chest medical image.

According to still another feature of the present disclosure, the processor may be further configured to overlap the class activation maps each other, and determine an average value of activation degrees for pixels of the overlapping class activation maps as an activation degree of the pixel of the entire class activation maps.

According to still another feature of the present disclosure, the processor may be further configured to overlap and display the rib fracture probability for each patch and the reconstructed entire class activation map with the entire chest medical image.

According to still another feature of the present disclosure, the convolutional neural network model may include one or more convolutional neural network models selected from ResNet, EfficientNet, VGGNet, GoogLeNet, LeNet, AlexNet, U-Net, OverFeat, DenseNet, and ZFNet.

According to still another feature of the present disclosure, the chest medical image may be at least one selected from an X-ray image, computed tomography (CT), magnetic resonance imaging (MRI), and positron emission tomography (PET).

### Effects of the Invention

The present disclosure may provide information that enables medical staff to quickly diagnose whether or not there is the rib fracture and the rib fracture point by visually illustrating the rib fracture point and the rib fracture probability from the chest medical image based on the artificial neural network model including the convolutional neural network using the patch as the input and the class activation map.

Through this, medical staff may quickly diagnose and treat the rib fracture even in emergency situations with other chest lesions, and save time required for diagnosing rib fractures.

Furthermore, the present disclosure may provide an environment in which medical staff may provide more appropriate treatment by reducing the rate of misdiagnosis.

The effects according to the present disclosure are not limited to the contents exemplified above, and more diverse effects are included in this document.

### Brief Description of Drawings

FIG. 1A is an exemplary illustration of a system for providing information on a rib fracture according to one embodiment of the present disclosure.
FIG. 1B is an exemplary illustration of a configuration of a device for providing information on the rib fracture according to one embodiment of the present disclosure.
FIG. 1C is an exemplary illustration of a configuration of a medical staff device that receives and outputs information on the rib fractures from the device for providing information on the rib fracture according to one embodiment of the present disclosure.
FIG. 2 is an exemplary illustration of a procedure of a method of providing information on the rib fracture according to one embodiment of the present disclosure.
FIG. 3 illustrates an example of a procedure for providing information on the rib fracture by reconstructing a class activation map classified into a class in which there is the rib fracture according to one embodiment of the present disclosure, to correspond to an entire chest medical image.
FIG. 4 illustrates a data set according to one embodiment of the present disclosure and characteristics of an evaluation model trained using the data set as input.
FIG. 5A to 5E illustrate evaluation results of an artificial neural network model according to one embodiment of the present disclosure.
FIG. 6 illustrates a chest medical image reconstructed with the artificial neural network model according to one embodiment of the present disclosure.

### Best Mode of the Invention

The advantages and features of the present disclosure, and the methods for achieving them, will become clearer with reference to the embodiments described in detail below together with the accompanying drawings. However, the present disclosure is not limited to the embodiments disclosed below, but may be implemented in various different forms, and these embodiments are provided only to make the disclosure of the present disclosure complete and to fully inform those skilled in the art of the scope of the disclosure, and the present disclosure is defined only by the scope of the claims. In connection with the description of the drawings, similar reference numerals may be used for similar components.

In this document, the expressions "have", "may have", "include", or "may include" indicate the presence of a feature (for example, a numerical value, function, operation, or component such as a part), but do not exclude the presence of additional features.

In this document, the expressions "A or B", "at least one of A and/or B", or "one or more of A or/and B" can include all possible combinations of the listed items. For example, "A or B", "at least one of A and B", or "at least one of A or B" may all refer to (1) including at least one A, (2) including at least one B, or (3) including both at least one A and at least one B.

The expressions "first", "second", or the like, used in this document may describe various components, regardless of order and/or importance, and are only used to distinguish one component from another, but do not limit the components. For example, a device of a first entity and a device of a second entity can represent devices of different entities, regardless of order or importance. For example, without departing from the scope of the rights set forth in this document, a first component can be named a second component, and similarly, a second component can also be renamed a first component.

When it is stated that a component (for example, a first component) is "(operatively or communicatively) coupled with/to" or "connected to" another component (for example, a second component), it should be understood that the component may be directly coupled to the other component, or may be connected via another component (for example, a third component). Conversely, when it is stated that a component (for example, a first component) is "directly coupled to" or "directly connected to" another component (for example, a second component), it should be understood that no other component (for example, a third component) exists between the component and the other component.

The expression "configured to (or set to)" as used herein may be used interchangeably with, for example, "suitable for", "having the capacity to", "designed to", "adapted to", "made to", or "capable of", depending on the situation. The term "configured to (or set to)" does not necessarily mean something that is "specifically designed to" in terms of hardware. Instead, in some contexts, the expression "a device configured to" may mean that the device is "capable of" doing something together with other devices or components. For example, the phrase "a processor configured (or set) to perform A, B, and C" may mean a dedicated processor (for example, an embedded processor) to perform those operations, or a generic-purpose processor (for example, a CPU or an application processor) that can perform those operations by executing one or more software programs stored in a memory device.

The terms used in this document are only used to describe specific embodiments and may not be intended to limit the scope of other embodiments. The singular expression may include the plural expression unless the context clearly indicates otherwise. The terms used herein, including technical or scientific terms, may have the same meaning as commonly understood by a person of ordinary skill in the art described in this document. Among the terms used in this document, terms defined in general dictionaries may be interpreted as having the same or similar meaning in the context of the related technology, and shall not be interpreted in an ideal or excessively formal meaning unless explicitly defined in this document. In some cases, even if a term is defined in this document, it cannot be interpreted to exclude the embodiments of this document.

The individual features of the various embodiments of the present disclosure may be partially or wholly combined or combined with each other, and as can be fully understood by those skilled in the art, various technical connections and operations are possible, and each embodiment may be implemented independently of each other or may be implemented together in a related relationship.

To ensure clarity in the interpretation of this document, the terms used in this document are defined below.

The term "subject" as used in this document may refer to any subject suspected of having a rib fracture. The subject disclosed in this document may be, but is not limited to, any mammal other than a human.

The term "chest medical image" as used in this document means an X-ray image, a computed tomography (CT), a magnetic resonance imaging (MRI), and a positron emission tomography (PET) that capture bone portions located in the chest, such as the sternum, ribs (rib bones), clavicle, spinal cord, and cervical vertebrae. Preferably, the chest medical image may mean the X-ray image. Meanwhile, "chest medical image" may mean multi-dimensional data composed of discrete image elements (for example, pixels in a two-dimensional image and voxels in a three-dimensional image), and may preferably be in the form of a digital imaging and ommunications in medicine (DICOM) file, which is an international standard for medical images.

The term "convolutional neural network (CNN) model" used in this document refers to a type of multilayer perceptron model that uses an image as input, classifies the input image, and outputs the classified image, and may be one or more models selected from ResNet, EfficientNet, VGGNet, GoogLeNet, LeNet, AlexNet, U-Net, OverFeat, DenseNet, and ZFNet, and may be further configured to an ensemble model that trains weights between the probabilities of training models generated by a plurality of task execution nodes.

The term "rib fracture" as used in this document means a fracture of one or more bones selected from the sternum, ribs (rib bones), clavicle, spinal cord, and cervical vertebrae located in the chest. Preferably, the rib fracture may mean the fracture of rib (rib bones).

The term "patch" used in this document may mean each of the divided squares when the entire chest medical image of a specific pixel size (for example, 1024 × 1024 pixels) is divided into square shapes of a specific size (for example, 256 × 256 pixels). Meanwhile, when the convolutional neural network model of the present disclosure extracts the patch image, the patch may extract duplicates from the entire chest medical image. At this time, the size of the extracted patch may be variable, and the convolutional neural network model of the present disclosure may have duplicate patch images as input.

The term "sliding window method" used in this document may mean a method in which, when selecting a region to extract the patch from the entire chest medical image in the "step of extracting the chest medical image patch by patch" of this document, patches are extracted sequentially from the four corners of the chest medical image in the left, right, and up and down directions rather than randomly, or each extracted patch is input into a model.

The term "class activation map" used in this document may include a major activation region with a high degree of activation determined by the convolutional neural network model of this document, and the major activation region may be normalized to have a value between 0 and 1 depending on the degree. Meanwhile, in this document, medical staff can check the area suspected of the rib fracture through the class activation map.

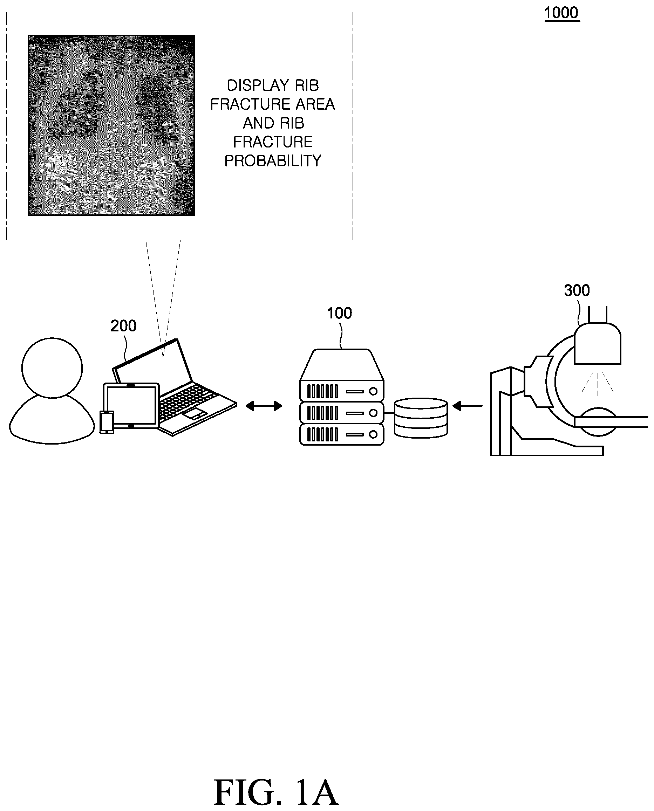

Hereinafter, a system for providing information on a rib fracture according to one embodiment of the present disclosure will be described with reference to FIGS. 1A to 1C.

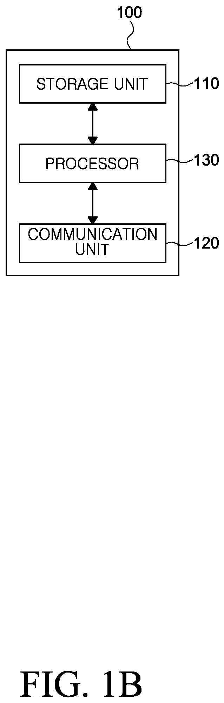

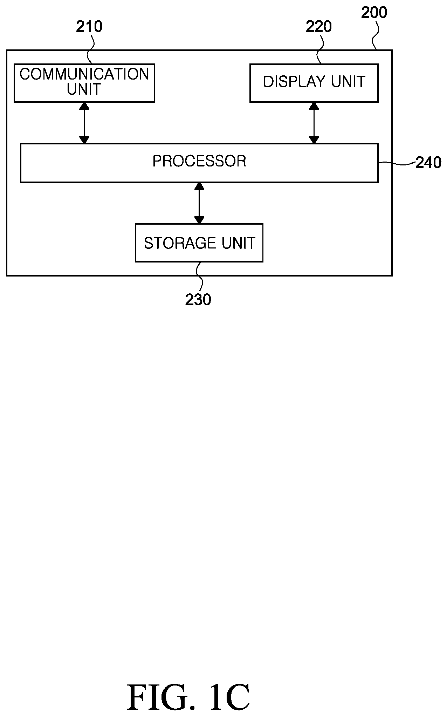

FIG. 1A exemplarily illustrates an information providing system for a device for providing information on the rib fracture according to one embodiment of the present disclosure. FIG. 1B illustrates an exemplary configuration of the device for providing information on the rib fracture according to one embodiment of the present disclosure. FIG. 1C illustrates an exemplary configuration of a medical staff device for receiving information on the rib fracture according to one embodiment of the present disclosure.

Referring to FIG. 1A, a system 1000 for information on the rib fracture may include a medical staff device 200, a device 100 for providing information on rib fracture, and a medical imaging device 300. Here, the medical staff device 200, the device 100 for providing information on rib fracture, and/or the medical imaging device 300 may be connected via a wired or wireless network.

First, the device 100 for providing information on rib fracture may include a general-purpose computer, laptop, and/or data server that performs various operations to provide information on rib fractures using chest medical image data transmitted from a medical imaging device 300, and may preferably be an edge computing server. Furthermore, by including a display unit, the information on rib fracture may be displayed to medical staff without a procedure of providing the information to a medical staff device 200.

Next, the medical staff device 200 may include at least one of a smartphone, a tablet personal computer (PC), a laptop, and/or a PC as an electronic device for displaying the information on rib fracture.

Finally, the medical imaging device 300 may be a medical device for taking an X-ray image, computed tomography (CT), magnetic resonance imaging (MRI), and positron emission tomography (PET) as a device for capturing the chest medical image.

Next, with reference to FIG. 1B, the components of the device 100 for providing information on rib fracture of the present disclosure will be specifically described.

Referring to FIG. 1B, the device 100 for providing information on rib fracture includes a storage unit 110, a communication unit 120, and a processor 130.

First, the storage unit 10 may store various data on the rib fracture of the subject. For example, the storage unit 10 may be configured to store data including patch images extracted by a convolutional neural network model and rib fracture probabilities for each patch. In various embodiments, the storage unit 10 may include at least one type of storage medium among flash memory type, hard disk type, multimedia card micro type, card type memory (for example, SD or XD memory, or the like), RAM, SRAM, ROM, EEPROM, PROM, magnetic memory, magnetic disk, and optical disk.

The communication unit 120 connects the device 100 for providing information on rib fracture to enable communication with an external device. The communication unit 120 may be connected to the medical staff device 200 and further, the medical imaging device 300 using wired/wireless communication to transmit and receive various data. Specifically, the communication unit 120 may receive the chest medical image of the subject from the medical imaging device 300. Furthermore, the communication unit 120 may transmit a diagnosis result to the medical staff device 200. Furthermore, by including the display unit, the information on rib fracture can be displayed to the medical staff without a procedure of providing the information to the medical staff device 200 (not illustrated).

The processor 130 is operably connected to the storage unit 110 and the communication unit 120 and can perform various commands for analyzing the chest medical images of the subject.

Specifically, the processor 130 may be configured to extract the chest medical image patch by patch based on the chest medical image received via the communication unit 120 and determine the rib fracture probability patch by patch. In this case, the processor 130 may be based on a pretrained convolutional neural network model to determine the rib fracture probability from the chest medical image patch image.

Furthermore, the processor 130 may be configured to generate a class activation map (CAM) patch by patch.

Furthermore, the processor 130 may reconstruct the entire class activation map generated patch by patch to correspond to the entire chest medical image.

Meanwhile, the device 100 for providing information on rib fracture is not limited to being designed in hardware. For example, the processor 130 of the device 100 for providing information on rib fracture may be implemented in software. Accordingly, the information on rib fracture may be displayed through the display unit of the medical imaging device 300 to which the software is connected.

Next, referring to FIG. 1C, the medical staff device 200 includes a communication unit 210, a display unit 220, a storage unit 230, and a processor 240.

The communication unit 210 may be configured to enable the medical staff device 200 to communicate with an external device. The communication unit 210 may be connected to the device 100 for providing information on the diagnosis of gallbladder polyps using wired/wireless communication and may transmit various data related to providing information on rib fractures. Specifically, the communication unit 210 may receive visual information related to a rib fracture of a subject received from the device 100 for providing information on rib fracture, but is not limited thereto.

The display unit 220 may display various interface screens for indicating information on rib fracture of the subject. For example, the display unit 220 may display the chest medical image of the subject, and further, may display a point predicted to be the rib fracture and the rib fracture probability at the point on the chest medical image. Furthermore, the class of patches classified by the convolutional neural network as a class in which there is the rib fracture may be highlighted to visually indicate a point predicted to be a rib fracture.

In various embodiments, the display unit 220 may include a touchscreen and may receive, for example, touch, gesture, proximity, drag, swipe, or hovering input using an electronic pen or a part of the user's body.

The storage unit 230 may store various data used to provide a user interface for displaying result data. In various embodiments, the storage unit 230 may include at least one type of storage medium among a flash memory type, a hard disk type, a multimedia card micro type, a card type memory (for example, an SD or XD memory, or the like), a andom access memory (RAM), a static random access memory (SRAM), a read-only memory (ROM), an electrically erasable programmable read-only memory (EEPROM), a programmable read-only memory (PROM), a magnetic memory, a magnetic disk, and an optical disk.

The processor 240 is operably connected to the communication unit 210, the display unit 220, and the storage unit 230, and may perform various commands to provide the user interface for displaying result data.

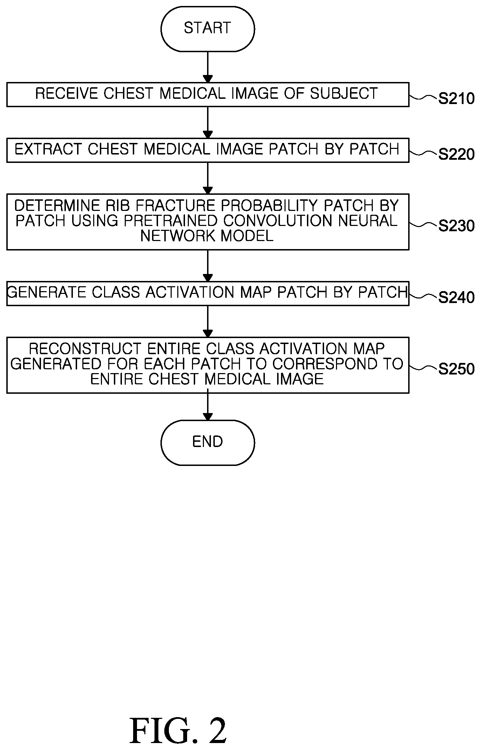

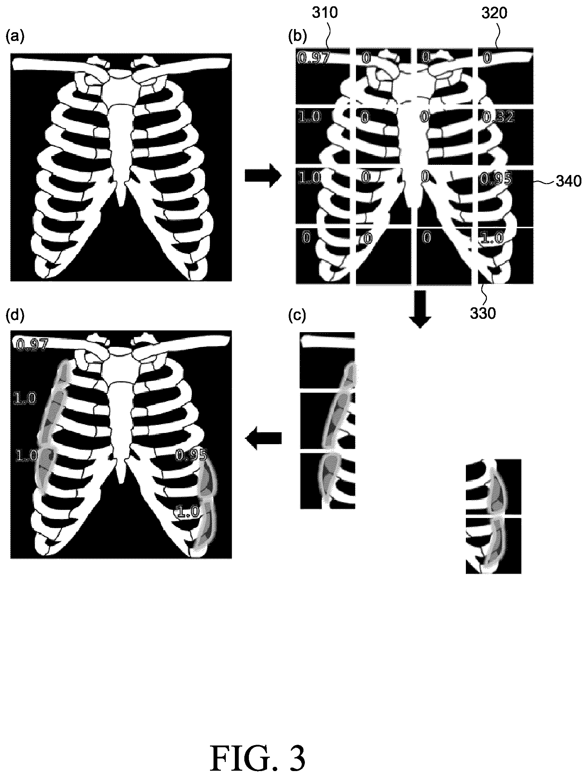

Hereinafter, a method of providing information on rib fracture according to one embodiment of the present disclosure will be specifically described with reference to FIGS. 2 and 3.

FIG. 2 illustrates an exemplary procedure of the method of providing information on rib fracture according to one embodiment of the present disclosure. FIG. 3 illustrates an exemplary procedure of the method of providing information on rib fracture, in which the class activation map classified into the class in which there is the rib fracture is reconstructed to correspond to an entire chest medical image according to one embodiment of the present disclosure. At this time, the drawing symbols described above in FIGS. 1A to 1C are used for convenience of explanation.

Referring to FIG. 2, the procedure of the method of providing information on rib fracture according to one embodiment of the present disclosure is as follows. First, the chest medical image of the subject is received (S210). Next, the chest medical image is extracted patch by patch (S220), and the rib fracture probability is determined for each patch using a convolutional neural network model pretrained to determine the rib fracture probability by using the patch as an input (S230). Next, the class activation map (CAM) is generated patch by patch (S240), and the generated entire class activation map is reconstructed to correspond to the entire chest medical image (S250).

More specifically, in the step (S210) of receiving the chest medical image of the subject, the processor 240 may receive one or more chest medical images selected from an X-ray image, computed tomography (CT), magnetic resonance imaging (MRI), and positron emission tomography (PET). Furthermore, the chest medical image received by the processor may mean a medical image in which bones are photographed regardless of the front, back, or side of the body.

Below, for the explanation of FIG. 2, FIG. 3 will be referred to together.

(a) of FIG. 3 exemplarily illustrates the step (S210) of receiving the entire chest medical image, (b) of FIG. 3 exemplarily illustrates the step (S230) of extracting the chest medical image patch by patch (S220) and then determining the rib fracture probability patch by patch, (c) of FIG. 3 exemplarily illustrates a step of generating the class activation map only in the patch having the rib fracture probability of 0.5 or higher, and finally, (d) of FIG. 3 exemplarily illustrates a step of overlapping and displaying the rib fracture probability value of the patch having the rib fracture probability of 0.5 or higher in the entire chest medical image and the class activation map generated in (c) of FIG. 3.

Referring to FIG. 2 and (a) of FIG. 3, after the step (S210) of receiving the chest medical image of the subject, the processor 240 may convert the entire chest medical image into a two-dimensional matrix value, and then convert the matrix value into an RGB file of a specific size (for example, 1024 × 1024 pixel size), and further convert (normalization) the RGB file into an output value between 0 and 1 through an activation function of a conventionally trained convolutional neural network model of the present disclosure.

Referring to FIG. 2 and (b) of FIG. 3, in the step (S220) of extracting the chest medical image patch by patch, the processor 240 may not randomly select the patch to be extracted from the entire chest medical image, but may extract the chest medical images while sequentially moving the chest medical images in a sliding window manner in the left, right, and up and down directions from the four corner ends of the chest medical image. Furthermore, in addition to the step (S220) of extracting patch by patch, the rib fracture probability patch by patch may be determined (S230).

For example, assuming that the size of the entire chest medical image of (a) of FIG. 3 is 1024 × 1024 pixels, (b) of FIG. 3 extracts patches of 256 × 256 pixels, and the numbers above each patch exemplarily represent the rib fracture probability. Furthermore, the number 0.97 of the patch 310 of (b) of FIG. 3 represents the fracture probability of 97%, 0 of the patch 320 represents 0%, 1 of the patch 330 represents 100%, and 0.32 of the patch 340 represents the rib fracture probability of 32%. However, the present disclosure is not limited thereto, and the size of the patch may vary, and the sliding may be configured such that the pixels of the patches overlap.

In various embodiments, in the step (S220) of extracting the chest medical image patch by patch and the step (S230) of determining the rib fracture probability patch by patch, the patches may be extracted while moving the sliding window by a length of 130 pixels smaller than the size of the patch, 260 × 260 pixels. Accordingly, the rib fracture probability of the patches whose extraction areas overlap may be determined as the average value or the maximum value of the rib fracture probabilities of the overlapping patches. Preferably, the average value of the rib fracture probabilities of the overlapping patches may be determined as the rib fracture probability for each patch.

Furthermore, in the step (S220) of extracting the chest medical image patch by patch, when extracting patches while moving the sliding window by a pixel length smaller than the patch size, in the step (S240) of generating the class activation map (CAM) patch by patch, the class activation maps for each patch overlap, and at this time, the average value of the activation degrees for the pixels of the overlapping class activation maps may be determined as the pixel activation degree for the overlapping class activation maps.

Next, referring to FIGS. 2, (c) of 3, and (d) of 3 together, in the step (S240) of generating the class activation map patch by patch, the class activation map may be generated for the entire patch regardless of the rib fracture probability, but the class activation map can also be generated only for patches having the rib fracture probability of 0.5 or higher. Furthermore, in the step (S250) of overlapping and reconstructing the generated class activation map according to the location information for each patch so that the class activation map corresponds to the entire chest medical image, the reconstructing may be performed using only patches having the rib fracture probability of 0.5 or higher. That is, when patches having the rib fracture probability of less than 0.5 are classified into the class activation maps, the activation degree for areas where there is no rib fracture may interfere with the determination of the user, so only the class activation maps of patches having the rib fracture probability of 0.5 or higher may be used to overlap and reconstruct the entire chest medical image ((d) of FIG. 3).

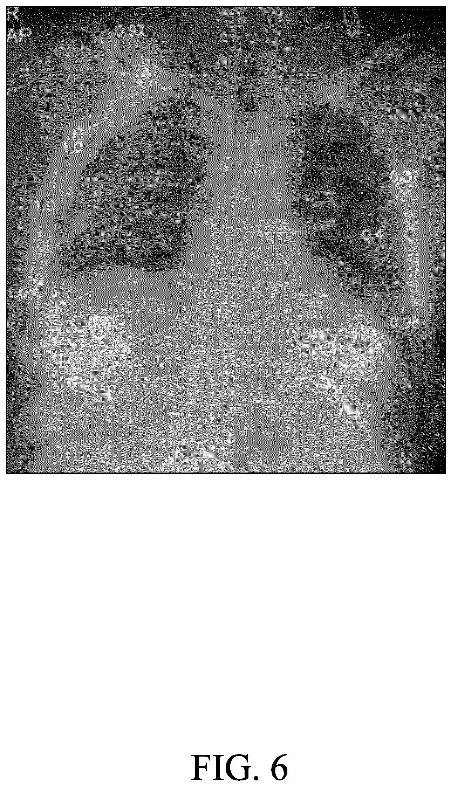

Furthermore, referring to (d) of FIG. 3, in the step (S250) of reconstructing the generated class activation map to correspond to the entire chest medical image, the rib fracture probability of each patch may be displayed within the region of each patch having the rib fracture probability of 0.5 or higher. Preferably, the rib fracture probability may be displayed within the activation region so that it can be recognized that the rib fracture probability is the rib fracture probability of the activation region of the class activation map generated from the patch having the rib fracture probability of 0.5 or higher. At this time, the rib fracture probability may be displayed in a format as in FIG. 3 and FIG. 6, may be expressed as a percentage, and may also be expressed as various images including a bar graph or diagram that may indicate accuracy.

This may reduce the time it takes for medical staff to directly examine the fracture site predicted by the artificial intelligence model predicting the rib fractures, and may also have the advantage of reducing the memory usage of the artificial intelligence model predicting rib fractures.

Meanwhile, the rib fracture probability of 0.5 determined as the threshold value is not a fixed value, and when a close examination of whether or not a rib fracture exists is required, the cutoff value for the rib fracture probability may be set to less than 0.5 in the step (230) of determining the rib fracture probability patch by patch, and the class activation map may be generated in patches above a specific value of less than 0.5 for the rib fracture probability to display the rib fracture prediction site.

FIG. 6 illustrates the chest medical image reconstructed using the artificial neural network model according to one embodiment of the present disclosure.

Referring to FIG. 6, when the processor 240 reconstructs the class activation map to correspond to the entire chest medical image, it may display the rib fracture probability for each patch so as to be overlapped with the entire chest medical image. Through this, medical staff may quickly diagnose whether there is the rib fracture and the rib fracture point by referring to the rib fracture point and the rib fracture probability from the chest medical image based on the artificial neural network model of the present disclosure. Furthermore, it is possible to provide an environment in which more appropriate treatment can be received by reducing the misdiagnosis rate through artificial intelligence.

### Evaluation: Evaluation of artificial neural network model used in embodiment of present disclosure

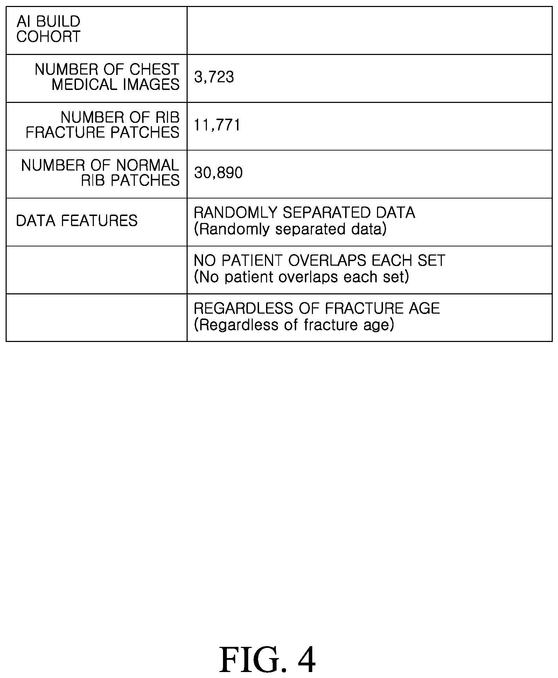

Hereinafter, data, model characteristics, and model validation results of the model of the present disclosure according to various embodiments of the present disclosure will be described with reference to FIGS. 4, 5A to 5E, and 6.

FIG. 4 illustrates the characteristics of a data set and the characteristics of an evaluation model trained using the data set as input according to one embodiment of the present disclosure.

Referring to FIG. 4, 3,723 chest medical images were collected to build the evaluation model, from which 11,771 rib fracture patches and 30,890 normal rib patches were obtained and used as a training data set. At this time, the data was randomly separated data, no patient overlaped in each data set, and there was regardless of fracture age.

Furthermore, in the model of the present disclosure, the input chest medical image was adjusted to 1024 × 1024 pixels, and the patch size extracted therefrom may be 260 × 260, 256 × 256, or 226 × 226 pixels. In the convolutional neural network model of the present disclosure, the training data set has undergone 5-fold validation, and may be set to 30 epochs per fold, 0.0003 as Lr, 36 as batch size, Adam as optimizer, and BCEWithLogitsLoss as loss function. Transpose, Vertical/Horizontal Flip, JpegCompression, ShiftCaleRotate, RandomBrightnessContrast, and HueSaturationValue may be performed for data expansion.

Furthermore, the shape of the sliding window during patch extraction and generation of the class activation map for each patch may be rectangular or square, and the size of the window may be various sizes of pixels, including 260 × 260, 256 × 256, or 224 × 224 pixels. The movement of the sliding window may be 130 pixels per step, and the sliding window movement distance may be variable to any pixel size.

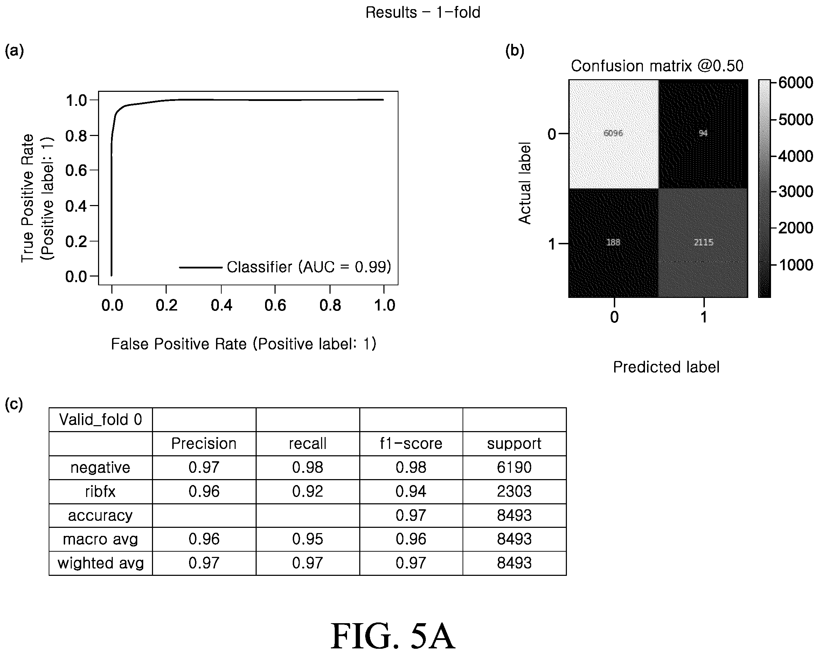

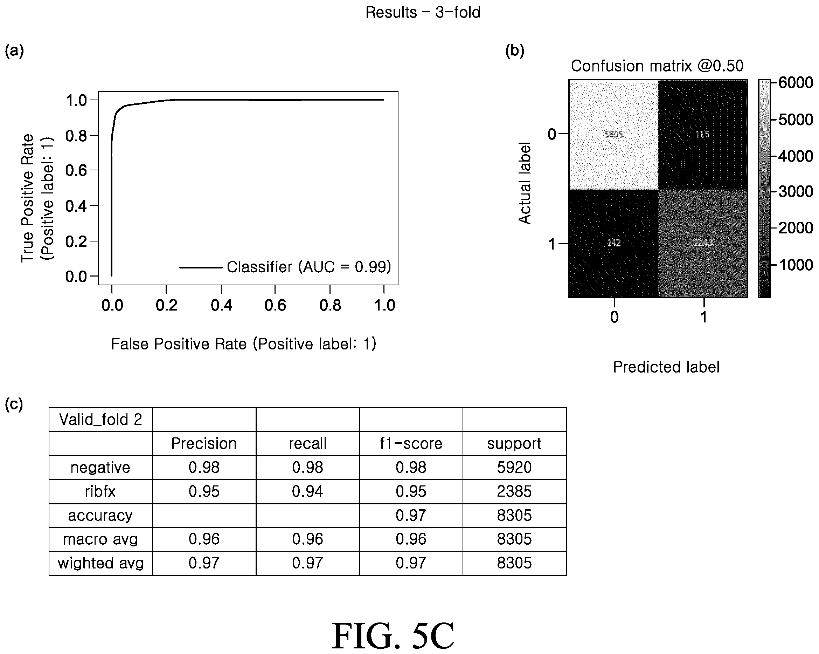

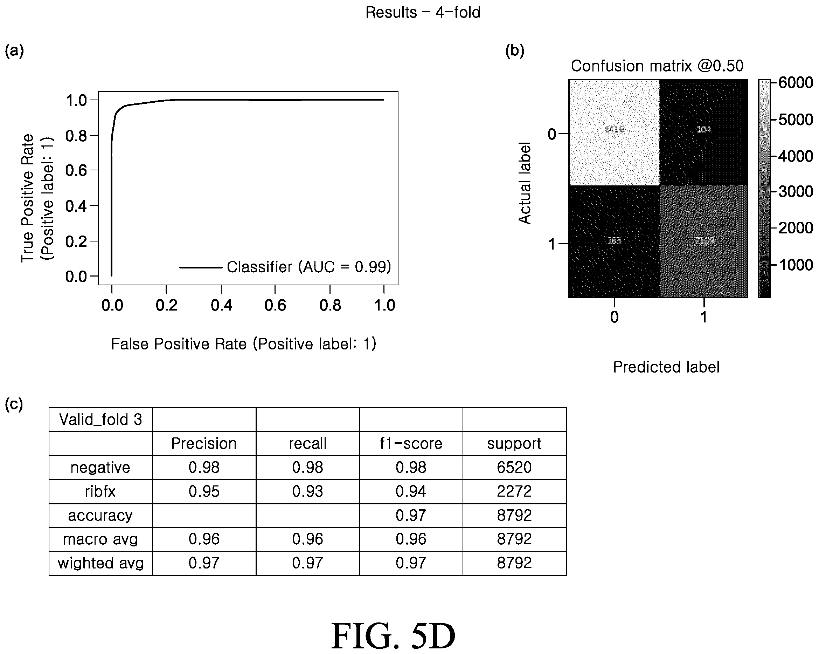

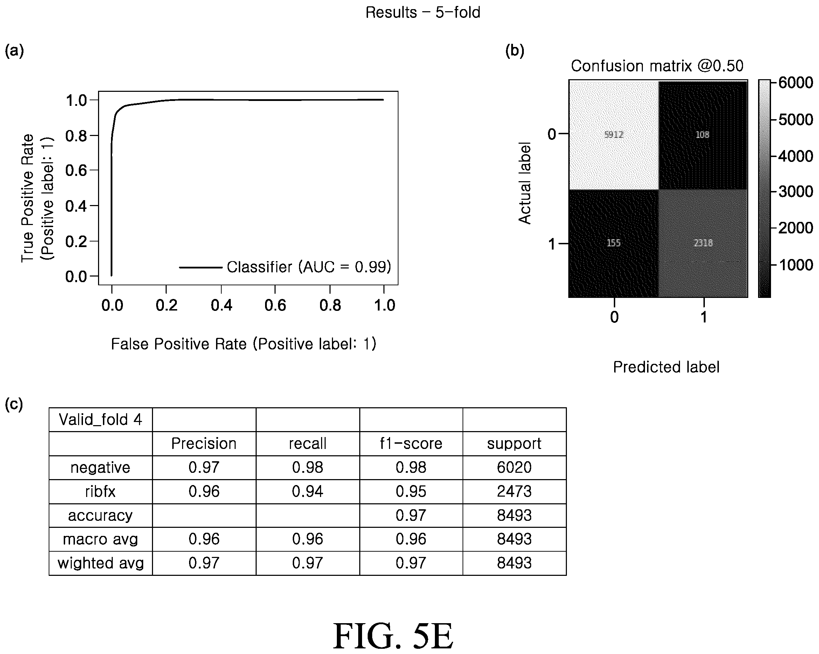

Referring to FIGS. 5A to 5E, the results of 5-fold validation of a training data set according to one embodiment of the present disclosure are illustrated.

Referring to the model validation results in FIG. 5A, the precision, recall, and F1-score for normal ribs (negative) are 0.97, 0.98, and 0.98, and the sensitivity, recall, and F1-score for rib fractures (ribfx) are also 0.96, 0.92, and 0.94. These results may mean that the evaluation model of the present disclosure has excellent performance and high reliability.

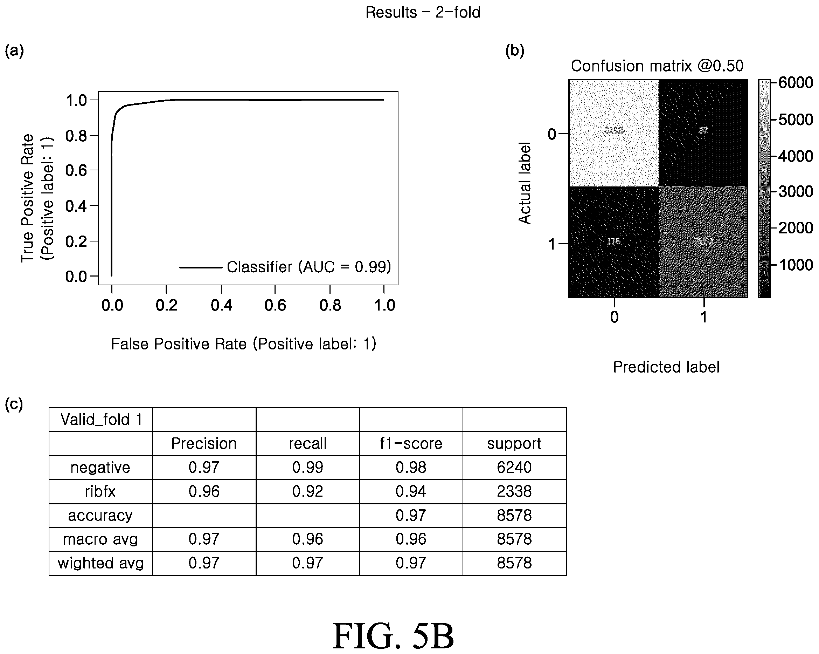

The model validation results of FIGS. 5B to 5E also illustrate that the performance and reliability of the evaluation model of the present disclosure are excellent, as the precision, recall, and F1-score for rib fractures show high values of 0.9 or higher, as in FIG. 5A.

Referring to FIG. 6, the chest medical image reconstructed with the rib fracture probability for each patch and the class activation map for each patch according to one embodiment of the present disclosure is illustrated.

Referring to FIG. 6, the rib fracture probability in the chest medical image is depicted as a number. Here, 1.0 means 100% rib fracture probability, and 0.47 means 47% rib fracture probability. Other numbers displayed in the chest medical image also indicate the rib fracture probability. Furthermore, the points indicated in red and yellow indicate points predicted to be rib fractures by reconstructing the class activation map of the present disclosure.

That is, the artificial neural network model for providing information on rib fracture of the present disclosure may illustrate the point expected to be a rib fracture and the rib fracture probability with high accuracy.

Although the embodiments of the present disclosure have been described in more detail with reference to the attached drawings, the present disclosure is not necessarily limited to these embodiments, and various modifications may be made without departing from the technical idea of the present disclosure. Accordingly, the embodiments disclosed in the present disclosure are not intended to limit the technical idea of the present disclosure, but to explain it, and the scope of the technical idea of the present disclosure is not limited by these embodiments. Therefore, it should be understood that the embodiments described above are exemplary in all aspects and not restrictive. The protection scope of the present disclosure should be interpreted by the following claims, and all technical ideas within a scope equivalent thereto should be interpreted as being included in the scope of the rights of the present disclosure.

### [Description of the symbol]

1000: System for providing information on rib fracture
100: Device for providing information on rib fracture
110, 230: Storage unit
120, 210: Communication unit
130, 240: Processor
200: Medical staff device
220: Display unit
300: Medical imaging device

[National Research and Development Projects Supporting the Disclosure]
[Project Identification Number] 1711088524
[Project Number] 2017R1A2B2011366
[Ministry] Ministry of Science and ICT
[Project Management (Specialized) Organization Name] National Research Foundation of Korea
[Research Project Name] Individual Basic Research (Ministry of Science and ICT) (R&D)
[Research Project Name] Research on Automated Detection in Chest X-ray Images Using Convolutional Neural Networks
[Contribution Rate] 1/1
[Project Executing Organization Name] Yonsei University
[Research Period] March 1, 2019 to February 29, 2020

## Claims

1. A method of providing information on a rib fracture, implemented by a processor, the method comprising:
receiving a chest medical image of a subject;
extracting the chest medical image patch by patch;
determining a rib fracture probability with a patch as input, using a convolutional neutral network model pretrained to determine the rib fracture probability by using the patch as an input;
generating a class activation map (CAM) patch by patch; and
reconstructing the entire class activation map generated patch by patch, to correspond to the entire chest medical image.

2. The method of claim 1, wherein the extracting of the chest medical image patch by patch further includes extracting the patch of a predetermined size while moving the patch in a sliding window manner.

3. The method of claim 1, wherein the determining of the rib fracture probability further includes determining, when extraction areas of the patches overlap each other, an average value or maximum value of the rib fracture probabilities of the overlapping patches as the rib fracture probability for each patch.

4. The method of claim 1, wherein the determining of the rib fracture probability further includes
classifying the class activation map into a class in which there is a rib fracture when the rib fracture probability is equal to or more than a predetermined value, and
classifying the class activation map into a class in which there is no rib fracture when the rib fracture probability is less than the predetermined value.

5. The method of claim 4, wherein the reconstructing of the entire class activation map so that the class activation map generated patch by patch corresponds to the entire chest medical image includes reconstructing, when reconstructing, only the class activation map classified as the class in which there is the rib fracture.

6. The method of claim 1, wherein the generating of the class activation map patch by patch further includes
overlapping the class activation maps each other, and
determining an average value of activation degrees for pixels of the overlapping class activation maps as an activation degree of the pixel of the overlapping class activation maps.

7. The method of claim 1, further comprising overlapping and displaying the rib fracture probability for each patch and the reconstructed entire class activation map with the entire chest medical image.

8. The method of claim 1, wherein the convolutional neural network model includes one or more convolutional neural network models selected from ResNet, EfficientNet, VGGNet, GoogLeNet, LeNet, AlexNet, U-Net, OverFeat, DenseNet, and ZFNet.

9. The method of claim 1, wherein the chest medical image is at least one selected from an X-ray image, computed tomography (CT), magnetic resonance imaging (MRI), and positron emission tomography (PET).

10. A device for providing information on rib fractures, the device comprising:
a communication unit configured to receive a chest medical image of a subject; and
a processor connected to communicate with the communication unit,
wherein the processor configured to
extract the chest medical image patch by patch,
determine rib fracture probability patch by patch by inputting the extracted chest medical image to a pretrained convolutional neural network model,
generate a class activation map (CAM) patch by patch, and
reconstruct the entire class activation map generated patch by patch, to correspond to the entire chest medical image.

11. The device of claim 10, wherein the processor is configured to extract a patch of a predetermined size while moving the patch in a sliding window manner.

12. The device of claim 10, wherein the processor is further configured to determine, when extraction areas of the patches overlap each other, an average value or maximum value of the rib fracture probabilities of the overlapping patches as the rib fracture probability for each patch.

13. The device of claim 10, wherein the processor is further configured to
classify the class activation map into a class in which there is a rib fracture when the rib fracture probability is equal to or more than a predetermined value, and
classify the class activation map into a class in which there is no rib fracture when the rib fracture probability is less than the predetermined value.

14. The device of claim 13, wherein the processor is further configured to reconstruct only the class activation map classified as the class in which there is the rib fracture when reconstructing to correspond to the entire chest medical image.

15. The device of claim 10, wherein the processor is further configured to
overlap the class activation maps each other, and
determine an average value of activation degrees for pixels of the overlapping class activation maps as an activation degree of the pixel of the entire class activation maps.

16. The device of claim 10, wherein the processor is further configured to overlap and display the rib fracture probability for each patch and the reconstructed entire class activation map with the entire chest medical image.

17. The device of claim 10, wherein the convolutional neural network model includes one or more convolutional neural network models selected from ResNet, EfficientNet, VGGNet, GoogLeNet, LeNet, AlexNet, U-Net, OverFeat, DenseNet, and ZFNet.

18. The device of claim 10, wherein the chest medical image is at least one selected from an X-ray image, computed tomography (CT), magnetic resonance imaging (MRI), and positron emission tomography (PET).
